Europäisches Patentamt

**European Patent Office**

**Office européen des brevets**

(11) Publication number:

**0 326 067**
**A2**

## (12) EUROPEAN PATENT APPLICATION

(21) Application number: **89101164.5**

(22) Date of filing: **24.01.89**

(51) Int. Cl.⁴: **A61K 37/02 , C07K 15/06**

(30) Priority: **26.01.88 US 148473**

(43) Date of publication of application:
**02.08.89 Bulletin 89/31**

(84) Designated Contracting States:
**AT BE CH DE ES FR GB GR IT LI LU NL SE**

(71) Applicant: **E.I. DU PONT DE NEMOURS AND COMPANY**
**1007 Market Street**
**Wilmington Delaware 19898(US)**

(72) Inventor: **Ackerman, Neil Richard**
**22 Foxhill Lane**
**Greenville Delaware 19807(US)**
Inventor: **Galbraith, William**
**14 N. Townview Lane**
**Newark Delaware 19711(US)**
Inventor: **Irr, Joseph David**
**.386 Briar Lane**
**Newark Delaware 19711(US)**
Inventor: **Jaffee, Bruce Donald**
**3206 Heathwood Road**
**Wilmington Delaware 19810(US)**
Inventor: **Lischwe, Michael Anthony**
**113 Sheldon Drive**
**Newark Delaware 19711(US)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) **Use of cyclophilin as an anti-inflammatory and immunomodulatory agent.**

(57) Use of cyclophilin in mammals as an anti-inflammatory and immunomodulatory agent, for example, to treat chronic inflammatory diseases and autoimmune diseases and to prevent rejection of tissue grafts and organ and bone marrow transplants. Pharmaceutical formulations containing cyclophilin for administration nasally, buccally, topically or by injection.

Bovine Cyclophilin / LP-3

FIG. 1

VNPTVFFDIAVDGEPLGRVSFELFADKVPK

TAENFRALSTGEKGFGYKGSCFHRIIPGFM

CQCGDFTRHNGTGGKSIYGEKFDDENFILK
E

HTGPGILSMANAGPNTNGSQFFICTAKTEW

LDGKHVVFGKVKEGMNIVEAMERFGSRNGK

TSKKITIADCGQI
LE

———— direct protein sequencing
— - — - amino acid composition

# USE OF CYCLOPHILIN AS AN ANTI-INFLAMMATORY AND IMMUNOMODULATORY AGENT

## Background of the Invention

Cyclophilin is a cytosolic protein present in a wide variety of tissues that binds the immunosuppressant drug cyclosporin A (Handschumacher et al., Science 226:544-547, 1984). Cyclophilin occurs in many biological species, including mushrooms, sponges and zucchini, as well as mammals. Its concentration varies from tissue to tissue and is elevated in certain cancer cells and in activated lymphocytes. The highest levels occur in tissues where cyclosporin A exhibits its efficacy (lymphocytes) or toxicity (kidney, brain) (Koletsky et al., J. Immunol. 137:1054-1059, 1986).

Cyclophilin has been isolated and purified from human thymus and bovine thymus cytosol (Harding et al., J. Biol. Chem. 261:8547, 1986). Major and minor isoforms of both bovine and human cyclophilin have been identified and found to have an apparent molecular weight of 17 kilodaltons (kDa). The major bovine isoform has been completely sequenced and found to have 163 amino acid residues and Mr = 17,737. The first 72 amino-terminal residues of the major human isoform have been determined and found to be identical with the major bovine cyclophilin isoform (ibid.).

Complementary DNA (cDNA) encoding human cyclophilin has been isolated from a cDNA library obtained by cloning in E. coli cDNA obtained by reverse transcription of messenger RNA (mRNA) isolated from induced cells of the leukemic T-cell line Jurkat (Haendler et al., EMBO J. 6:947, 1987). The cDNA was sequenced and the amino acid sequence of human cyclophilin was deduced from the nucleotide sequence of the cDNA. The deduced sequence differed from the sequence of bovine thymus cyclophilin in only three respects, additional of a glutamic acid residue at the carboxyl terminus, substitution of leucine for isoleucine at residue number 163, and substitution of glutamic acid for aspartic acid at residue number 83. Southern blot analysis of genomic DNA prepared from human peripheral blood leukocytes indicated the existence of several genes coding for several isoforms of cyclophilin (ibid.)

Cyclosporin A is a cyclic undecapeptide immunosuppressant which is used to prevent rejection of tissue grafts and organ and bone marrow transplants. It is under investigation for treatment of autoimmune diseases. Although cyclophilin is known to bind cyclosporin A, it is not yet known what role, if any, cyclophilin plays in the immunosuppressive action of cyclosporin A (ibid.). There have been no reports in the literature of in vivo pharmaceutical utility of cyclophilin.

## Summary of the Invention

This invention is based on the discovery that cyclophilin exhibits potent in vivo anti-inflammatory activity in mice in a contact sensitivity test described in detail below. The test involves a delayed-type hypersensitivity reaction and is a reliable indicator of anti-inflammatory and immunomodulatory activity in humans and other mammals. It was unexpected and surprising to discover that cyclophilin exhibits similar activity to its binding ligand, cyclosporin A.

In one aspect, this invention is a method of reducing inflammation in a mammal having a chronic inflammatory disease or other inflammation, or modulating the immune system of a mammal having an autoimmune disease, tissue graft, or organ or bone marrow transplant, which comprises administering to the mammal an anti-inflammatory or immunomodulatory effective amount of cyclophilin.

In another aspect, this invention is a pharmaceutical formulation for intravenous (i.v.), intramuscular (i.m.), subcutaneous (s.c.), intraperitoneal (i.p.), nasal, buccal or topical administration consisting essentially of cyclophilin in an anti-inflammatory or immunomodulatory effective amount and a pharmaceutical carrier. Preferred is a formulation for i.v., i.m., s.c. or i.p. administration consisting essentially of cyclophilin in isotonic aqueous solution of a salt, buffer or sugar. The invention includes lyophilized cyclophilin in a sterile vial, suitable for reconstitution in such an isotonic aqueous solution to provide a formulation for i.m., i.v., s.c. or i.p. injection.

The invention includes a purified human cyclophilin isoform, designated LP3', never before isolated which has substantially the same amino acid composition and the same sequence in the first 42 amino acids of the amino-terminus as the previously-reported major bovine and human isoforms, except that threonine, alanine and aspartic acid are substituted for alanine, valine and glycine at positions 10, 11 and 13, respectively. Also included are blocked analogs of the new isoform, in which valine at position 1 is acetylated or preceded by an acetylated methionyl residue. These newly-isolated, purified isoforms are substantially free of other cell matter. Preferably, they are at least 95% pure, more preferably at least 99% pure.

The term "cyclophilin" as used herein means a naturally occurring cytosolic protein having a molecular weight of about 17 kilodaltons, which is capable of binding cyclosporin, and which exhibits anti-inflammatory activity in the animal model described below. It includes the previously-reported major and minor bovine and human isoforms, the various isoforms LP3, LP5, LP6, LP3', LP5' and LP6' isolated from U-937 cells as described herein, and biologically equivalent proteins which have substantially the same amino acid composition and sequence (70% or greater sequence homology) as the above isoforms.

The claims of this application are intended to encompass equivalents of cyclophilin, whether produced from cell isolates or by synthetic or recombinant means, such as polypeptides which differ from cyclophilin by the absence of post-translational mammalian modifications, and deletion, insertion and substitution mutants of cyclophilin, which bind cyclosporin A and exhibit anti-inflammatory activity in the animal model described below.

## Brief Description of the Drawing

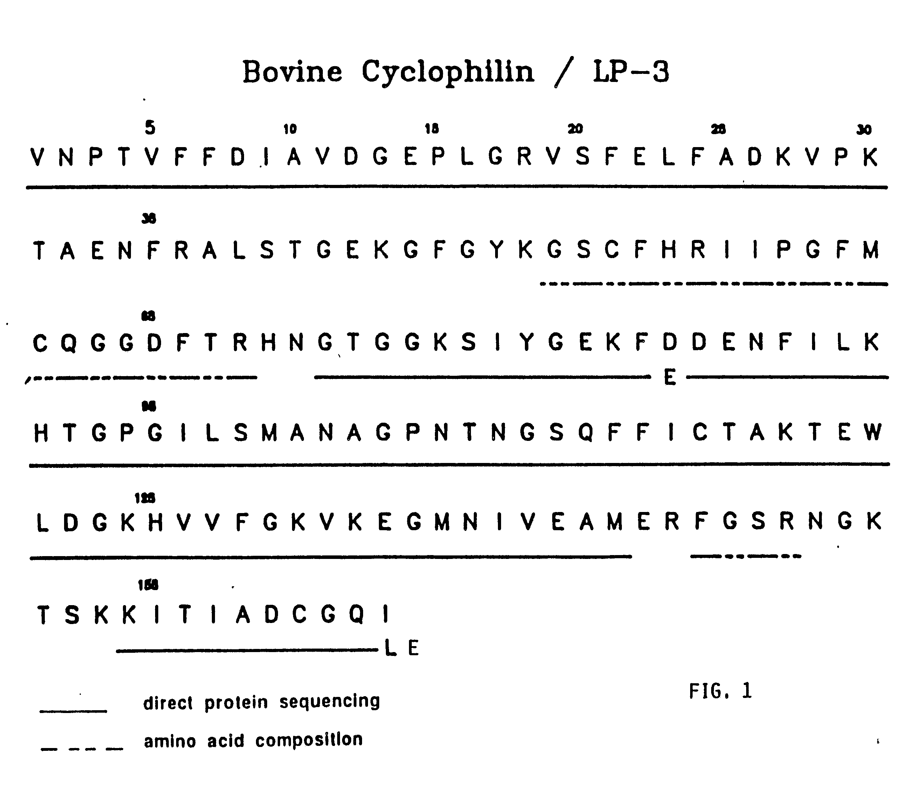

Figure 1 shows the previously reported amino acid (a.a.) sequence of bovine thymus cyclophilin (Harding et al., supra) and the differences between that and the a.a. sequence of isoform LP3 (described below) of cyclophilin which was isolated from a sub-clone of U-937 cells. The one letter and three letter symbols used for the amino acids are listed in Lehninger, Biochemistry, 2d Ed., p. 72, Worth Publishers, NY (1975). The lines below the a.a. sequence for bovine cyclophilin indicate the portions of LP3 which have been sequenced or analyzed for a.a. composition. E below D at a.a. position 83 indicates a change from aspartic acid to glutamic acid. L below I at position 163 indicates a change from isoleucine to leucine. In addition LP3 has an additional a.a., glutamic acid (E), at the carboxyl terminus. Thus, it appears that LP3 corresponds in a.a. sequence to the deduced sequence for Jurkat derived human cyclophilin reported in Haendler et al., supra.

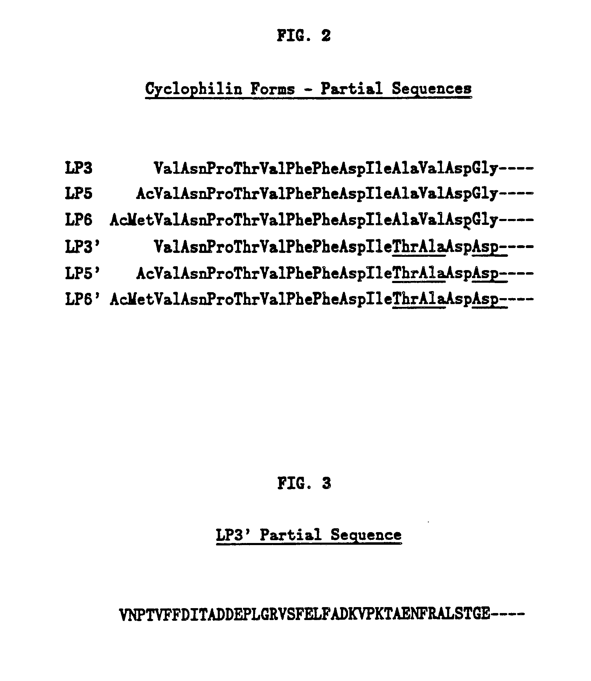

Figure 2, shows partial a.a. sequences for LP3 and other cyclophilin isoforms LP5, LP6, LP3', LP5' and LP6' isolated from the sub-clones of U-937 as described below. LP5 differs from LP3 in having an acetyl group (Ac) on the valine residue at the amino terminus. LP6 differs from LP3 in having an acetylated methionyl residue preceding valine at the amino terminus. LP3' differs from LP3 in having threonine, alanine and aspartic acid instead of alanine, valine and glycine at a.a. positions 10, 11 and 13, respectively. It is believed that LP5' and LP6' differ from LP3' in the same way that LP5 and LP6 differ from LP3.

Figure 3 shows the a.a. sequence for the first 42 a.a.'s of LP3'.

## Detailed Description of the Invention

Cyclophilin from any source can be used in the invention. For human use, it is preferred to use human cyclophilin to avoid possible adverse immune reaction. Based on the wide distribution of cyclophilin in several human tissues (Koletsky et al., supra) and the report of mRNA for cyclophilin from human Jurkat cells (Haendler et al., supra) it could be reasonably expected to isolate cyclophilin from many different leukocytic cell lines. In the tests reported below, the cyclophilin used was isolated and purified from a sub-clone of a human histocytic lymphoma cell line U-937 which was obtained and is publicly available from American Type Culture Collection, 12301 Parklawn Drive, Rockville, MD 20852, under ATCC accession number CRL-1593. The cloning, isolation and purification methods which were used are described in the Examples below.

Biological activity of cyclophilin in the Examples below was determined with an in vivo contact sensitivity test system using dinitrofluorobenzene (DNFB) as the sensitizer on mice in the system described by Phanuphak et al. (J. Immunol. 112:115, 1974).

BALB/c female mice (~20g, Charles River) were sensitized on the shaved abdomen with 25 µl of 0.5% DNFB in a vehicle of 4:1 acetone:olive oil on days 0 and 1. Mice were ear challenged with 20 µl of 0.2% DNFB in a vehicle of 4:1 acetone:olive oil on day 5. A constant area of the ears were measured immediately before challenge and 24 hours later with an engineer's micrometer. One hour prior to challenge (day 5) the groups received subcutaneous administration of compound in saline. Percent suppression was calculated

as:

$$\% \text{ suppression} = 1 - \frac{\text{compound treated-negative control}}{\text{positive control-negative control}} \times 100$$

Positive controls received only saline as the drug injection. Negative controls were not sensitized on days 0 and 1 but were challenged on day 5 and dosed with an injection of saline. Six to ten mice were used per group.

Example 1

A. Growth of U-937 Cells and Preparation of Extract

The U-937 cells were grown in Dulbecco's Modified Eagle Medium with 4.5 g/l glucose supplemented with 3.7 g/l sodium bicarbonate and 5-10% heat inactivated (56°C for 30 min) fetal bovine serum. The growth conditions were 37°C with 5% $CO_2$ in the air and 30 RPM stirring. The cells were harvested at a cell density of $72.5 \times 10^6$ cells/ml. A cell paste was prepared using a Sharples centrifuge. The cell paste was washed twice with phosphate buffered saline (Gibco #310-4040) supplemented with gentamycin (50 $\mu$g/ml). The cell paste can be stored frozen (-20°C) in Tris buffer (0.05 M, pH 8.1). Cells ($4 \times 10^{10}$) were brought to 200 ml with Tris buffer and sonicated in 25 ml aliquots using an Ultrasonics Sonicator (Model W375 with cup horn attachment) under the following conditions: a) 45 sec at maximum output, continuous pulse, b) hold on ice bath for 10 min., c) 60 sec at maximum output, 50% pulse. The cell sonicate was centrifuged 45,000 Xg for 60 min. and the supernatant used for further purification.

B. DEAE Column Purification

The protein concentration of the sonicated extract from the U-937 cells was determined by the Bradford procedure. The protein concentration varied from 8-12 mg/ml. The material was applied to a DEAE cellulose (diethylaminoethyl cellulose) column. The column was equilibrated in 0.05 M Tris•HCl pH 8.1. The maximum ratio of protein to resin used was 2.85 $\mu$g/ml. The flow rate was 1 ml/min. and 10 ml fractions were collected. The absorbance at 280 nm was monitored. The DEAE flow-through fractions were collected and pooled. Typically 10 to 20% of the protein applied did not bind to the column in 0.05 M Tris•HCl pH 8.1.

C. Alkaline Phosphatase Treatment

The DEAE flow-through fraction was next treated with alkaline phosphatase. The alkaline phosphatase used was attached to beaded agarose (Sigma Chemical Company, #P-0762). The alkaline phosphatase was equilibrated in 0.05 M Tris•HCl pH 8.1 before mixing with the protein solution. The incubation was for 15 minutes at 23°C with shaking. The ratio of alkaline phosphatase to protein used was 25 units/gram. After the incubation the suspension was passed through a Bio-Rad econocolumn to separate the alkaline phosphatase from the protein solution. The sample was sterile filtered and further processed or stored at 4°C.

D. HPLC Column Purification

The alkaline phosphatase treated protein sample was applied directly to a Vydac C4 HPLC column or the pH was lowered and then the sample was applied to the C4 column. The solvents used in the HPLC system were water and acetonitrile both containing 0.1% trifluoroacetic acid (TFA). The proteins were eluted from the C4 column with an acetonitrile gradient. Proteins of 17,000 molecular weight (p17), as determined by SDS-polyacrylamide gel electrophoresis, eluted at an acetonitrile concentration of from 35-40%. If the sample was treated with 20 mM dithiothreitol (DTT) for 20 minutes at room temperature before the application to the C4 column, three p17-containing peaks were evident. If the sample was not treated with DTT at least six peaks containing a predominant p17 were evident. DTT reduced the six or more peaks to three. The peaks containing the p17s were individually collected and dried.

The p17-containing peak fractions from the C4 column were individually dissolved in 0.1% TFA with or without 20 mM DTT. The fractions which contained DTT were incubated for 20 minutes at 23°C. The dissolved proteins were individually applied to a Vydac phenyl HPLC column. The p17s were determined to be greater than 95% pure after the phenyl column. The dried samples were dissolved as described above.

The p17-containing peak fractions from the phenyl column were individually applied to a Vydac C18 column. The p17s eluted at an acetonitrile concentration of from 37-41%. The three p17s, designated LP3, LP5 and LP6 were judged to be greated than 99% pure. The ratio of LP3:LP5:LP6 was approximately 2.6:1:2.1.

Purity was judged by HPLC elution profile and by polyacrylamide gel electrophoresis (PAGE) under reducing conditions in the presence of sodium dodecylsulfate (SDS). Comparison to molecular weight standards run on the SDS•PAGE gels gave the estimated 17 kDa (kilodalton) molecular weight. Amino acid analysis of the purified 17 kDa protein agreed with the published amino acid content of cyclophilin, and amino acid sequencing of LP3 from the amino terminus and of tryptic and hydroxylamine peptides (Figure 1) gave an exact match with the sequence predicted for human cyclophilin (Haendler et al., supra) for the 70% of the amino acids that were sequenced. The three distinct p17s represent the unblocked protein and protein with blocking groups acetyl and acetylmethionyl on the amino terminus, as shown in Fig. 2.

The determination of amino acid composition in this and subsequent Examples was carried out as follows. The samples were hydrolyzed for 24, 48 and 72 hours at 110°C with 6N HCl containing 0.4% 2-mercaptoethanol under an argon atmosphere. The resultant amino acids were resuspended in sodium citrate buffer pH 2.2 with norleucine added as an internal standard and identified on a Beckman 6300 analyzer (ion-exchange, post-column ninhydrin derivatized) (Beckman Instruments Spinco Division, Palo Alto, CA). The proteins were sequenced by Edman degradation on an Applied Biosystems Model 470A gas-phase sequencer with on-line Model PTH analyzer (Applied Biosystems Inc., Foster City, CA) as described by Hewick, et al. J. Biol. Chem. 256:7990, (1981). For preparing tryptic peptide maps, the reverse phase purified cyclophilin was treated with trypsin at a 1 to 50 (wt/wt) ratio for 24 hours at 23°C. The peptides were resolved on a C4 column using a Hewlett-Packard 1090M HPLC. The HPLC reagents were 0.1% TFA and 0.1% TFA/acetonitrile.

LP3, LP5 and LP6 were individually prepared for storage by adding mannitol and freeze drying. Enough mannitol was added to make the concentration 0.3 M when the protein concentration of the resolubilized material was made 1 mg/ml.

The freeze-dried preparations were dissolved in sterile, pyrogen-free water at a protein concentration of 1 mg/ml. This solution was diluted in sterile phosphate buffered saline to give the indicated doses in a total volume of 0.2 ml. The cyclophilin was given subcutaneously one hour before topical administration of a challenging dose of DNFB (0.2%) on day 5 of the contact sensitivity protocol described above. The ear swelling was measured on day 6. The activity was compared to the standard glucocorticosteroid, dexamethasone, and to the cyclophilin species heated at >90°C for 45 minutes (LP3hi, LP5hi, LP6hi) to destroy biological activity and provide a protein control.

| Treatment | Dose (μmg/mouse) | Ear Swelling[a] (units ± SEM) | % Suppression |
|---|---|---|---|
| Neg. control | saline | 1.2 ± 0.8 | --- |
| Pos. control | saline | 80.3 ± 4.7 | 0 |
| Dexamethasone | 2.0 | 66.0 ± 2.1 | 18.0 |
| | 20.0 | 52.0 ± 2.4 | 35.7 |
| | 200.0 | 21.3 ± 2.5 | 74.6 |
| LP3 | 0.001 | 57.0 ± 1.2 | 29.4 |
| | 0.01 | 45.7 ± 2.6 | 43.7 |
| | 0.1 | 35.0 ± 2.3 | 57.2 |
| | 1.0 | 41.8 ± 1.5 | 48.7 |
| LP5 | 0.0001 | 52.3 ± 3.2 | 35.3 |
| | 0.001 | 35.5 ± 2.0 | 56.6 |
| | 0.01 | 41.1 ± 3.7 | 49.5 |
| | 0.1 | 28.7 ± 1.4 | 65.2 |
| | 1.0 | 30.0 ± 1.4 | 63.6 |
| LP6 | 0.0001 | 57.0 ± 2.4 | 29.4 |
| | 0.001 | 58.7 ± 1.7 | 27.3 |
| | 0.01 | 40.2 ± 3.4 | 50.7 |
| | 0.1 | 35.6 ± 1.5 | 56.5 |
| | 1.0 | 30.1 ± 2.0 | 63.4 |
| LP3hi | 1.0 | 63.6 ± 1.4 | 21.1 |
| LP5hi | 1.0 | 67.0 ± 3.4 | 16.8 |
| LP6hi | 0.1 | 59.8 ± 2.3 | 25.9 |
| | 1.0 | 57.9 ± 2.1 | 28.3 |

[a] increase in ear thickness from day 5 to day 6 measured as 1 unit = $10^{-4}$ inches.

Example 2

The DEAE flow through fraction obtained as in Example 1, parts A-C, was treated with 20 mM DTT at room temperature for 20 minutes, the applied to a Vydac C18 column on the HPLC. The solvents used were 0.1% trifluoroacetic acid (TFA) and acetonitrile/0.1% TFA. The proteins were eluted from the column with an acetonitrile gradient. Three cyclophilin species designated LP3, LP5 and LP6 eluted from the column at between 37 and 42% acetonitrile. After the C18 column the proteins were greater than 90% pure.

In some experiments the p17-containing peaks from the C18 column were freeze dried, then solubilized in 0.1% TFA before application to a Vydac phenyl column. In other instances the peaks were diluted 1:2 in 0.1% TFA and applied to the phenyl column. The proteins eluted slightly sooner from the phenyl column. The three cyclophilin species were judged to be greater than 95% pure after the phenyl column. The p17-containing peak fractions from the phenyl column were then further purified using as a final chromatographic step a Vydac C4 column. The p17 products were determined to be greater than 99% pure by SDS•PAGE following the C4 column chromatography step. Changing the order of the three reverse phase columns above had little effect on the purity of the resulting proteins. The individual species were pooled in a ratio of 1:1:1 and designated LC15.

The pooled proteins were prepared for storage by adding mannitol and freeze drying. Enough mannitol was added to make the concentration 0.3 M when the protein concentration of the resolubilized material was made 1 mg/ml.

The freeze dried preparation was dissolved in sterile, pyrogen-free $H_2O$ at a protein concentration of 1 mg/ml. This solution was diluted in sterile phosphate buffered saline to give the indicated doses in a total volume of 0.2 ml. The cyclophilin was given subcutaneously one hour before topical administration of a challenging dose of DNFB (0.2 %) on day 5 of the contact sensitivity protocol described above. The ear swelling was measured on day 6. The activity was compared to the standard glucocorticosteroid, dexamethasone, and to cyclophilin heated at >90° C for 45 minutes (LC15hi) to destroy biological activity and thus act as a protein control.

| Treatment | Dose (μg/mouse) | Ear Swelling* (units ± SEM) | % Suppression |
|---|---|---|---|
| Negative | Saline | 4.00 ± 1.0 | |
| Positive | Saline | 81.30 ± 2.7 | 0 |
| Dexamethasone | 2.0 | 71.25 ± 1.6 | 13 |
| Dexamethasone | 20.0 | 56.30 ± 2.2 | 32 |
| Dexamethasone | 200.0 | 16.35 ± 2.7 | 84 |
| LC15 | 0.0001 | 52.56 ± 2.3 | 37 |
| LC15 | 0.001 | 38.13 ± 2.6 | 56 |
| LC15 | 0.01 | 45.85 ± 2.6 | 46 |
| LC15 | 0.1 | 58.35 ± 2.4 | 30 |
| LC15 | 1. | 48.70 ± 2.9 | 42 |
| LC15 | 10. | 59.55 ± 2.4 | 28 |
| LC15hi | 0.0001 | 66.90 ± 3.7 | 19 |
| LC15hi | 0.001 | 64.90 ± 3.2 | 21 |
| LC15hi | 0.01 | 70.5 ± 3.2 | 14 |
| LC15hi | 0.1 | 61.70 ± 4.3 | 25 |
| LC15hi | 1. | 60.20 ± 4.5 | 27 |
| LC15hi | 10. | 65.85 ± 4.4 | 20 |

* increase in ear thickness from day 5 to day 6 measured as 1 unit = $10^{-4}$ inches.

## Example 3

The DEAE flow through fraction obtained as in Example 1, Parts A-C, was dialyzed against 10 mM potassium phosphate pH 7.2/5 mM DTT. This sample was applied to an Amicon Matrex Gel Blue A column equilibrated in the same buffer. Most of the contaminating proteins were eluted from the column with 100 mM potassium phosphate pH 7.2/5 mM DTT. The cyclophilin was eluted with 350 mM potassium phosphate pH 7.2/5 mM DTT. The high salt wash-off was dialyzed against 5 mM potassium phosphate pH 6.8 and applied to a Du Pont weak cation exchange HPLC column (WCX-HPLC). The proteins were eluted with a sodium chloride gradient of from 0 to 0.5 M in 5 mM potassium phosphate pH 6.8. The proteins were concentrated with a second Blue A column and the buffer was exchanged in an Amicon spinning cell. The resulting cyclophilin was greater than 95% pure, was at 1.0 mg/ml in phosphate buffered saline, and contained less than 10 endotoxin units/mg.

This solution was diluted in sterile phosphate buffered saline to give the indicated doses in a total volume of 0.2 ml. The cyclophilin was given subcutaneously one hour prior to topical administration of a challenging dose of DNFB (0.2%) on day 5 of the contact sensitivity protocol. The ear swelling was measured on day 6. The activity was compared to the standard glucocorticosteroid, dexamethasone, and to cyclophilin heated at >90°C for 45 minutes (cyclophilin hi) to destroy biological activity and thus act as a protein control.

7

| TREATMENT | DOSE (μg/MOUSE) | Δ EAR SWELLING[a] (UNITS ± SEM) | % SUPPRESSION |
|---|---|---|---|
| Negative Control | saline | 5.8 ± 0.7 | - |
| Positive Control | saline | 73.4 ± 4.0 | 0 |
| Dexamethasone | 2.0 | 56.8 ± 4.3 | 24.6 |
| | 20.0 | 38.2 ± 2.8 | 52.1. |
| | 200.0 | 3.5 ± 2.8 | 103.4 |
| Cyclophilin | 0.001 | 50.8 ± 2.8 | 33.4 |
| | 0.01 | 47.5 ± 2.8 | 38.4 |
| | 0.1 | 46.3 ± 4.0 | 40.1 |
| | 1.0 | 45.8 ± 3.6 | 40.8 |
| | 10.0 | 41.5 ± 3.7 | 47.2 |
| | 100.0 | 42.3 ± 3.1 | 46.1 |
| Cyclophilin hi | 0.001 | 62.1 ± 4.7 | 16.7 |
| | 0.01 | 58.5 ± 4.0 | 22.1 |
| | 0.1 | 66.3 ± 4.0 | 10.5 |
| | 1.0 | 56.0 ± 4.6 | 25.7 |
| | 10.0 | 72.5 ± 3.8 | 1.3 |
| | 100.0 | 60.3 ± 4.1 | 19.4 |

* increase in ear thickness from day 5 to day 6 measured as 1 unit = $10^{-4}$ inches.

## Example 4

The U-937 cell line was treated with BMCyclin (Boehringer Mannheim) to remove mycoplasma. The mycoplasma-free cells were then subcloned and the subcloned cells were sonicated as described in Example 1, Part A, then the sonicate was applied to a DEAE cellulose column as described in Example 1, Part B. The DEAE flow through fraction was applied to an Amicon Matrex Gel Blue A column and a WCX-HPLC column as described in Example 3. In addition to LP3, LP5 and LP6 which eluted at 0.12 M NaCl, three new cyclophilin species were eluted from the WCX-HPLC column at 0.05 M NaCl. The new species, designated LP3', LP5' and LP6', were shown to bind cyclosporin and are expected to have activity in the mouse contact sensitivity test, and thus be useful as anti-inflammatory and immunomodulatory agents. LP5' was not separated from LP6' by the procedure of Example 3, but could be separated by the procedures of Examples 1 and 2. LP3' was substantially identical to LP3 in a.a. composition and the first 42 a.a.'s at the amino-terminus were identical in sequence to LP3 except at positions 10, 11 and 13, as shown in Fig. 2. LP5' and LP6' were shown to be amino-terminus blocked forms, and it is believed that they are identical to LP3' except that the valine at position 1 is acetylated in LP5' and preceded by acetylated methionyl in LP6'. The ratio of LP3':LP5':LP6' was about 1:1:1.6.

## Doses and Formulations

The contact sensitivity test system described above is a delayed-type hypersensitivity reaction; similar inflammatory reactions occur in humans. Non-steroidal anti-inflammatory drugs such as aspirin and ibuprofen which do not suppress the immune system do not inhibit the mouse contact sensitivity reaction. Methotrexate, cyclosporin A, and the glucocorticosteroids such as dexamethasone suppress the immune system, inhibit the mouse contact sensitivity reaction, and have utility in treating human chronic inflammation. Results in this model indicate that cyclophilin is useful for treating inflammation, especially chronic inflammatory diseases such as rheumatoid arthitis, psoriasis, chronic inflammatory bowel disease, and asthma. Based on its wide range of efficacious doses in acute studies with the mouse contact sensitivity model (0.05 μg/kg to 5 mg/kg) cyclophilin may have an advantage over other compounds which inhibit the model but are also toxic at higher doses. Results in the model also indicate that cyclophilin should be useful in preventing tissue graft rejection and organ and bone marrow transplant rejection. In addition, it should be useful in treating autoimmune diseases, such as rheumatoid arthritis, systemic lupus erythematosus, multiple sclerosis, myasthenea gravis and juvenile diabetes, in view of the knowledge that major T lymphocyte components are found in both these disease states and in the contact sensitivity reaction.

Based on the activity in the mouse contact sensitivity test, doses in the range of 0.5 to 5,000 micrograms per kilogram of body weight would be anti-inflammatory and immunomodulatory effective amounts of cyclophilin. For a 70 kg adult human, therefore, doses is in the range of 35 micrograms to 350 milligrams would be used when the drug is administered topically or by injection. Doses at the lower end of this range, e.g., 0.1 to 10 mg would be used for nasal or buccal administration.

For administration the cyclophilin should be mixed with a pharmaceutically suitable carrier. The term "carrier" in this context means one or more other ingredients which are substantially biologically inert and which render the drug more tolerable for the patient and/or more available to the patient. The concentration of cyclophilin in the carrier should be sufficient to provide an anti-inflammatory or immunomodulatory effective amount without an unduly large volume dose. Ordinarily, the concentration will be in the range of about 0.1 to 5 mg/ml for an injectable formulation and in the range of about 0.1 to 5% for topical, nasal and buccal formulations. However, higher and lower concentrations can be used. For i.v., i.m., s.c., and i.p. administration the cyclophilin should be dispersed in an isotonic aqueous solution of a salt, buffer or sugar. A cyclophilin solution can be lyophilized in sterile vials and reconstituted immediately prior to administration by addition of sterile water for injection. For nasal, topical and buccal administration, the cyclophilin should be mixed with absorption enhancers and may also be mixed with dispersing agents and/ or solubilizers. For suitable pharmaceutical carriers reference is made to Remington's Pharmaceutical Sciences, Fourteenth Edition, Mack Publishing Co., Easton, PA (1970). The following are examples of formulations of the invention, but the invention is not limited to these particular formulations.

Example 5

Injectable Liquid Dosage Form

1 mg of cyclophilin is dissolved in a buffer solution containing 0.01 M sodium phosphate, 0.15 M NaCl and a selected preservative to provide a total volume of 1 ml. The pH of the resulting solution is adjusted to 7.2 with NaOH or HCl. The final solution is then aseptically filtered and filled into sterile containers for parenteral use.

Example 6

Freeze Dried Powder

1 mg of cyclophilin is dissolved in a 0.3 M mannitol solution in water to provide a total volume of 1 ml and the pH is adjusted to 7.2 with NaOH or HCl. The final solution is then aseptically filtered and filled into sterile vials which are partially stoppered with butyl rubber stoppers. Lyophilization is carried out with primary drying temperature at 20°C and secondary drying temperature at 40°C. The freeze-dried vials are stoppered under nitrogen and sealed immediately. The finished vials can be reconstituted with enough water to provide 1 mg/ml of cyclophilin and to make a clear and isotonic solution for parenteral use.

Example 7

| Nasal Formulation : (metered spray to deliver 0.1 cc) | |
|---|---|
| Cyclophilin | 0.1-5 % |
| Sodium Cholate (absorption enhancer) | 0.1 |
| Methylparaben (Preservative) | 0.05 |
| Purified Water | 94.85-99.75 |

Example 8

| Buccal Patch | |
|---|---|
| Cyclophilin | 0.1-5 % |
| Sodium Cholate | 0.1 |
| Methylparaben (Preservative) | 0.05 |
| PEG 400 | 3.0 |
| CarbopolTM 934P | 5.0 |
| KlucelTM EF | 86.85-91.75 |

Other possible absorption enhancers which can be used in addition to or instead of sodium cholate include sodium taurocholate, sodium glycocholate, sodium deoxycholate, sodium taurodeoxycholate, and sodium glycodeoxycholate.

Claims

1. A method of reducing inflammation in a mammal having a chronic inflammatory disease or other inflammation, or modulating the immune system of a mammal having an autoimmune disease, tissue graft, or organ or bone marrow transplant, which comprises administering to the mammal an anti-inflammatory or immunomodulatory effective amount of cyclophilin.

2. A pharmaceutical formulation for intravenous, intramuscular, subcutaneous, intraperitoneal, nasal, topical or buccal administration consisting essentially of cyclophilin in an anti-inflammatory or immunomodulatory effective amount and a pharmaceutically suitable carrier.

3. Pharmaceutical formation of claim 2 for intravenous, intramuscular, subcutaneous or intraperitoneal administration consisting essentially of cyclophilin in isotonic aqueous solution.

4. Lyophilized cyclophilin in a sterile vial, suitable for reconstitution with sterile water to provide a pharmaceutical formulation of claim 3.

5. A cyclosporin A binding, human cytosolic protein having a molecular weight of about 17 kDa, having a purity of at least about 95% by weight, and having the amino acid sequence
VNPTVFFDITADDEPLGRVSFELFADKVPKTAENFRALSTGE
at positions 1-42, or a blocked analog thereof having the same amino acid sequence and purity, wherein valine at position 1 is acetylated or preceded by an acetylated methionyl residue.

6. An acetylated, cyclosporin A binding, human cytosolic protein having a purity of at least about 95% by weight and the amino acid sequence
VNPTVFFDIAVDGEPLGRVSFELFADKVPKTAENFRALSTGE KGFGYKGSCFHRIIPGFMCQGGDFTRHNGTG-
GKSIYGEKFED ENFILKHTGPGILSMANAGPNTNGSQFFICTAKTEWLDGKHV VFGKVKEGMNIVEAMERFGS-
RNGKTSKKITIADCGQLE
wherein V at position 1 is acetylated or preceded by an acetylated methionyl residue.

# Bovine Cyclophilin / LP-3

```
        5              10             18             20             28             30
V N P T V F F D I A V D G E P L G R V S F E L F A D K V P K
_____

T A E N F R A L S T G E K G F G Y K G S C F H R I I P G F M
                                      - - - - - - - - - - - - - - - - - - -

C Q G G D F T R H N G T G G K S I Y G E K F D D E N F I L K
,- - - - - - - - - -         _____ E _____

H T G P G I L S M A N A G P N T N G S Q F F I C T A K T E W
_____

L D G K H V V F G K V K E G M N I V E A M E R F G S R N G K
_____      - - - - - - -

T S K K I T I A D C G Q I
_____ L E
```

_____   direct protein sequencing

_ __ _   amino acid composition

FIG. 1

EP 0 326 067 A2

# FIG. 2

## Cyclophilin Forms - Partial Sequences

LP3      ValAsnProThrValPhePheAspIleAlaValAspGly----

LP5      AcValAsnProThrValPhePheAspIleAlaValAspGly----

LP6  AcMetValAsnProThrValPhePheAspIleAlaValAspGly----

LP3'     ValAsnProThrValPhePheAspIleThrAlaAspAsp----

LP5'   AcValAsnProThrValPhePheAspIleThrAlaAspAsp----

LP6' AcMetValAsnProThrValPhePheAspIleThrAlaAspAsp----

# FIG. 3

## LP3' Partial Sequence

VNPTVFFDITADDEPLGRVSFELFADKVPKTAENFRALSTGE----